# EUROPEAN PATENT SPECIFICATION

(11) **EP 0 309 411 B1**
(45) Date of publication and mention of the grant of the patent: **02.03.1994**
(21) Application number: 88810646.5
(22) Date of filing: 21.09.1988
(51) Int. Cl.: C07H 13/06, A61K 31/70

(54) **Saccharide derivatives**
Saccharid-Derivate
Dérivés de saccharides

(30) Priority: 23.09.1987 DE 3731953
(43) Date of publication of application: 29.03.1989
(73) Proprietor: SANDOZ AG, 4002 Basel (CH); SANDOZ-PATENT-GMBH, 79539 Lörrach (DE); SANDOZ-ERFINDUNGEN Verwaltungsgesellschaft m.b.H., 1235 Wien (AT)
(72) Inventor: Macher, Ingolf, A-6250 Breitenbach (AT)

(56) References cited:
- EP-A- 0 224 260
- WO-A-84/04526
- WO-A-86/05687
- MEDICAL MICROBIOLOGY AND IMMUNOLOGY, 1986, pages 5-8, Washington, DC, US; K. TAKAYAMA et al.: "Structures of lipid A, its precursors, and derivatives"
- INFECTION AND IMMUNITY, vol. 55, no. 11, November 1987, pages 2636-2644, Washington, DC, US; L. BRADE et al.: "The immunogenicity and antigenicity of lipid A are influenced by its physicochemical state and environment"
- CHEMISTRY LETTERS, no. 8, August 1982, pages 1281-1284, Tokyo, JP; M. INAGE et al.: "A convenient preparative method of carbohydrate phosphates with butyllithium and phosphorochloridate"
- AGRICULTURAL AND BIOLOGICAL CHEMISTRY, vol. 45, no. 2, 1981, pages 545-548, Tokyo, JP; M. KISO et al.: "Synthesis of 2-(acylamino)-2-deoxy-alpha-D-glucopyranosyl phosphates: monosaccharide analogs of lipid A"
- INFECTION AND IMMUNITY, vol. 45, no. 1, July 1984, pages 293-296, Washington, DC, US; S. KOTANI et al.: "Immunobiologically active lipid A analogs synthesized according to a revised structural model of natural lipid A"
- J. Biol. Chem. 1990, 265, 9159-64
- Infect. Immun 1983, 41, 758-73
- Infect. Immun. 1991, 59, 2351-8
- Antimicrob.Agents Chemother 1991,35,500-5

## Description

The present invention relates to new saccharide derivatives, namely new α-D-glucopyranose derivatives.

The invention comprises the compounds of formula I 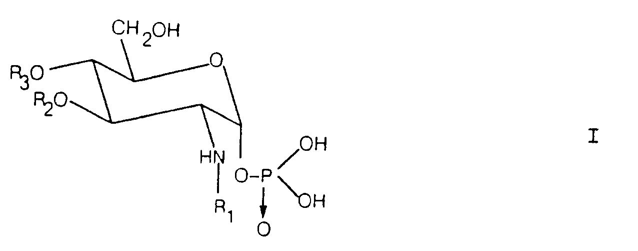
wherein
- R₁, R₂ and R₃: independently are alkylcarbonyl of altogether 12 to 16 carbon atoms optionally monosubstituted in the 3 position by hydroxy or alkylcarbonyloxy, whereby the alkylcarbonyl part of alkylcarbonyloxy is of altogether 12 to 16 carbon atoms,
in free form or in salt form.

The ring structure has the configuration of α-D-glucopyranose.

Alkylcarbonyl and the alkylcarbonyl part of alkylcarbonyloxy preferably are of altogether 14 carbon atoms. The carbon atom in the 3 position, when substituted, may have the R or S configuration. In the corresponding compounds of formula I R₁, R₂ and R₃ may thus be in achiral form or in the R or S configuration. When they are in chiral form the R configuration is preferred.

The configuration remains unchanged when the processes described herein are effected, namely, when R, S or racemic compounds are used as starting material, the corresponding R, S, or, respectively, racemic compounds are obtained.

Alkylcarbonyl preferably is substituted.

Preferred substituent of alkylcarbonyl is hydroxy.

R₁, R₂ and R₃ preferably are identical.

A subgroup of compounds of formula I is the compounds of formula I wherein R₁, R₂ and R₃ are as defined above after formula I with the proviso that R₂ and R₃ are identical, in free form or in acid addition salt form. In this subgroup R₁ may or may not be identical to R₂ and R₃.

A further subgroup of compounds of formula I is the compounds of formula I wherein R₁, R₂ and R₃ are as defined above after formula I with the proviso that when R₁ is 3-hydroxydodecanoyl or 3-hydroxyhexadecanoyl, then at least one of R₂ and R₃ is other than dodecanoyl or 3-(R)-hydroxydodecanoyl. In a subgroup thereof R₁, R₂ and R₃ are as defined above after formula I with the proviso that R₁ is other than 3-hydroxydodecanoyl or 3-hydroxyhexadecanoyl and R₂ and R₃ are other than dodecanoyl or 3-hydroxydodecanoyl.

A further subgroup of compounds of formula I is the compounds of formula I wherein R₂ and R₃ are as defined above after formula I and R₁ is alkylcarbonyl of altogether 12 to 16 carbon atoms optionally monosubstituted in the 3 position by hydroxy. In a subgroup thereof R₁ and R₂ are alkylcarbonyl of altogether 12 to 16 carbon atoms optionally monosubstituted in the 3 position by hydroxy. In a subgroup thereof R₁, R₂ and R₃ are alkylcarbonyl of altogether 12 to 16 carbon atoms optionally monosubstituted in the 3 position by hydroxy. In a subgroup thereof R₁, R₂ and R₃ are alkylcarbonyl of altogether 12 to 16 carbon atoms monosubstituted in the 3 position by hydroxy.

The structure of the compounds of the invention is somewhat related to that of lipid A of gram-(-)-bacteria and its monosaccharide precursor, lipid X, and to the compounds disclosed in e.g. the following publications:
Sandoz EP 180 608
WARF WO 86/05687
Sandoz WO 87/00174.

However, before the date of this invention, 2,3,4-triacylated glucosamines had never been described as components or derivatives of lipid A. The very broad and speculative formula disclosed on page 5 in WARF WO 86/05687 might be argued to encompass 2,3,4-tri- and 2,3,4,6-tetraacylated glucosamines but this disclosure provides no method to obtain such compounds, which do not occur naturally. The compounds of the present invention moreover possess particularly beneficial pharmacological properties.

The invention also provides a process for the preparation of a compound of formula I in free form or in salt form comprising deprotecting a corresponding compound of formula II 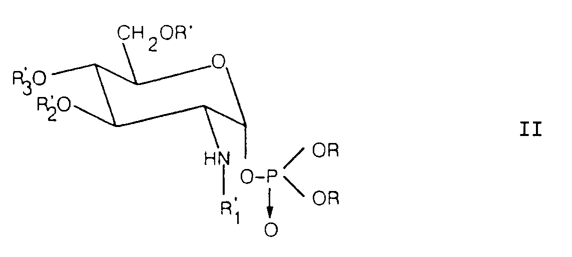
wherein
R and R' are protecting groups and
R₁', R₂' and R₃' have the significance indicated above for, respectively, R₁, R₂ and R₃ with the proviso that any hydroxy group therein is in protected form,
and recovering the resultant compound of formula I in free form or in salt form.

The process of the invention is a deprotection reaction. It may be effected in accordance with known methods. Depending on the nature of the protecting groups deprotection may be effected in one single step or in several successive steps. When the protecting groups are hydrogenolytically splittable groups such as benzyl or triphenylmethyl the deprotection may e.g. be effected hydrogenolytically, e.g. by hydrogenating over palladium on charcoal.

Indicated as protecting groups are protecting groups generally used in saccharide chemistry. R' may e.g. be the triphenylmethyl group. Indicated protecting groups R for the phosphate or hydroxy group are also generally known, e.g. benzyl.

The resultant compounds of formula I may be recovered from the reaction mixture and purified in accordance with known methods.

The compounds of formula I may be recovered in free form or in salt form. Preferred salt forms are e.g. salts with basic hydrophilic compounds such as tris(hydroxymethyl)aminomethane and L-lysine. Salt forms may be obtained from the free base forms in known manner, and vice-versa.

The compounds used as starting materials may be obtained in accordance with known procedures.

The compounds of formula II may be obtained e.g. by appropriately acylating corresponding compounds of formula III 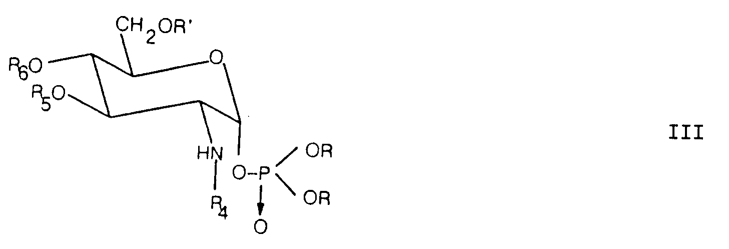
wherein
- R and R': are as defined above and
- R₄, R₅ and R₆: are hydrogen or have the significance indicated above for, respectively, R₁, R₂ and R₃ with the proviso that any hydroxy group therein is in protected form and the further proviso that at least one of R₄, R₅ and R₆ is hydrogen.

The acylation is preferably effected at a reduced temperature, e.g. at about 4°C. The reaction is preferably effected in an inert solvent such as a hydrocarbon, e.g. methylene chloride. The acylating agent may e.g. be supplemented with dicyclohexylcarbodiimide and 4-dimethylaminopyridine.

The compounds of formula III may be obtained e.g. starting from the compound of formula IV 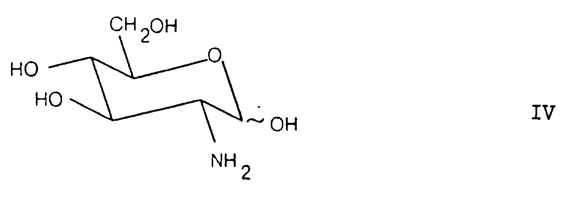
by appropriately protecting and substituting the OH and NH₂ groups therein using the methods generally known in the chemistry of saccharides. The choice of the protecting groups is effected depending on the position of the corresponding OH and NH₂ groups, while any OH or, respectively, NH₂ group which it is foreseen to acylate in the following step is left unprotected. A first acylation step to acylate e.g. the NH₂ and/or an OH group is followed by a deprotection step. The deprotection step is itself followed by a selective protection step leaving one or more OH groups unprotected. Subsequently acylation may again be effected, if appropriate with another acyl group. Repetition of similar reaction steps allows the preparation of compounds wherein the three acyl groups are different. In formula IV the configuration of the OH group in the 1 position is indifferent, introduction of the phosphate group leads to compounds having the configuration indicated in formula III at the corresponding position.

It should be noted that in any event for each acylation step the OH group in the 6 position must be protected since it is not intended to acylate it. The same also applies for the phosphate residue, which must be protected prior to each acylation step. The phosphate residue may e.g. be introduced by reaction of an organometallic compound such as butyl lithium with the compound of formula IV or a partially acylated derivative thereof optionally having the hydroxy group in position 6 in protected form, and thereafter adding e.g. dibenzylphosphorochloridate to the reaction mixture. The reaction with the organometallic compound preferably is effected in an inert solvent, e.g. in a cyclic ether such as tetrahydrofuran. The reaction temperature preferably is reduced, e.g. -50°C. The solvent is an aliphatic hydrocarbon such as hexan. The hydroxy groups of the phosphate residue are protected, e.g. by benzyl.

Insofar as the preparation of the starting materials is not particularly described herein, these are known or may be prepared in accordance with known procedures or in a manner analogous to known procedures, e.g. analogous to the procedures described in the Examples.

The compounds of formula I in free form or in pharmaceutically acceptable salt form, hereinafter referred to as the compounds of the invention, possess pharmacological activity. They are therefore indicated for use as pharmaceuticals.

In particular, the compounds of the invention exert an effect on the unspecific antimicrobial resistance. This appears from e.g. the following tests:
1. Determination of endotoxic activity in the limulusamoebocytes lysate test.
2. Induction of endotoxin shock in the mouse.
3. Microbial septicemia in the neutropenic mouse (number of germs per mouse, e.g. Pseudomonas aeruginosa Δ12: about 6x10⁴; E.coli Δ120: about 2 x 10⁶; Staph. aureus Δ113: about 2 x 10⁶; Candida albicans Δ124: about 2 x 10⁴).
   The compounds of the invention effect marked improvements in time and rate of survival over untreated infection controls in experimental infections with gramnegative agents (e.g. Pseudomonas and E.coli) as well as in infections with grampositive agents (e.g. Staph.aureus) or yeasts (e.g. Candida albicans), after parenteral administration.
4. Activation of the oxidative metabolism of human blood neutrophils.
5. Carbon clearance test.
6. Herpes infection in the mouse.
   In the experimental HSV-1-infection, the compounds of the invention cause marked improvements as regards the course of illness, survival time and survival rate over untreated controls. These effects are observed after a single i.p. or s.c. administration between days 0 or -1 and +6.
7. CSF induction (colony-stimulating factor).
   The compounds of the invention induce CSFs to various extents in mice, whereby time-kinetic differences in CSF-activities are also observed. These could be advantageous in therapeutic use.
8. Induction of Interleukin-1 (IL-1).
   The compounds of the invention possess to various extents (in concentrations of 0.1 - 50 µg/ml) the capacity to induce IL-1 production in macrophages.
9. Induction of LPS- (endotoxin) tolerance (lethality tolerance).
   The compounds of the invention are eliciting this tolerance already after single i.p. administration of 0.25 mg/mouse. Pretreated (tolerant) mice are exposed to a LPS-challenge at a dosage of 0.01 µg LPS + 8 mg galactosamine/mouse i.p. at various times (1 day to 3 weeks) following the last treatment. A larger proportion of animals survive this LPS challenge, especially after repeated administration (3 times), as compared with challenge controls not pretreated.

Furthermore, the compounds of the invention possess antiinflammatory activity, especially in nonspecific, in immunologically-induced, and in hypersensitivity-induced inflammation and in allergic reactions.

This activity may be demonstrated by various test methods, e.g. by investigation of the influence on prostaglandin synthesis in vitro and in vivo. A marked inhibition of LPS- or zymosan-induced PGE₂-production is found. The inhibition of LPS-induced PGE₂-release by mouse peritoneal leucocytes after pretreatment with test substances is measured in vivo and a marked reduction in PGE₂-release is found as compared with controls.

In a further test, the influence on procoagulant activity (PCA) is measured.

The addition of test substance reduces the PCA elicited by LPS as compared to the control value as manifested by an increase in coagulation time. Pretreatment with test substance similarly results in reduction of PCA.

In vivo, the influence of LPS-induced PCA by mouse peritoneal leucocytes after pretreatment with test substance may be shown. The PCA of rabbit peritoneal leucocytes may be reduced after induction of the generalized Shwartzman reaction, and by pretreatment with LPS or the substance, respectively.

An almost complete inhibition of Shwartzman-reaction is observed after pretreatment with LPS or test substance, respectively.

Furthermore, the compounds of formula I possess activity against tumors, as demonstrated in the following tests:
1. Induction of tumor necrosis factor (TNF)
2. B16F1 melanoma test

It is found that the compounds of the invention possess immunoprophylactic activity, which reflects in a reduction of the number of B16F1 melanoma metastases in the lungs. As a test of therapeutic activity, mice are treated on day 3, 6, 8, 10, 13 and 15 after inoculation of the tumor cells. Here, too, a marked reduction in the number of metastases is observed.

The description of the pharmacological test methods referred to above has been published in WO 87/00174.

The compounds of the invention are therefore indicated for use as modulators of unspecific antimicrobial resistance, in the systemic enhancement of immune response, and in the enhancement of unspecific immunity. The compounds of the invention are thus useful in the treatment or supportive treatment (i.e. together with other specific or supportive therapeutic forms) of conditions associated with decreased immune response, especially decreased humoral immune response and/or decreased over-sensitivity reaction of the delayed type, and in the treatment of conditions in which generally a modulation of immune response is indicated. In particular, the compounds of the invention are useful in the treatment or supportive treatment of pathological conditions based on idiopathic immunological deficiencies or immunological deficiencies of the type encountered in geriatric patients, or in patients with heavy burns or generalized infections. The compounds of the invention are also useful in the treatment or supportive treatment of viral illnesses (such as disseminated Herpes, progressive smallpox and disseminated varicella diseases), of Morbus Hodgkin and other malignant tumors. Furthermore, the compounds of the invention are indicated for use in the prevention of endotoxin shock, e.g. in accidents, burns, and surgical interventions, and as antiinflammatory agents.

For the above-mentioned uses the dosage will, of course, vary depending on the compound employed, mode of administration and treatment desired. In general satisfactory results are obtained when administered at a daily dosage, or as a unique administration for the achievement of an adjuvant effect, e.g. in supportive treatment, of from about 0.0015 mg/kg to about 1 mg/kg animal body weight. Administration is e.g. parenterally, preferably i.p. For the larger mammal an indicated total daily dosage is in the range of from about 0.1 mg to about 70 mg, conveniently given, in the case of a daily dosage, in divided doses 2 to 4 times a day in unit dosage form containing from about 0.025 to about 35 mg of the compounds admixed or in sustained release form. An indicated total single adjuvant dosage is in the range of up to 70 mg of the compounds.

In view of their immunomodulating activity the compounds of the invention are also indicated for use as adjuvants in vaccines. For this use an indicated dosage is from about 0.5 mg to about 100 mg, preferably about 70 mg, administered on the day of vaccination with appropriately a repetition in the same dosage 2 to 4 weeks thereafter.

The invention further also provides pharmaceutical compositions comprising a compound of the invention in association with at least one pharmaceutical carrier or diluent, such compositions may be prepared in conventional manner, e.g. by mixing with conventional, pharmaceutically acceptable carriers or diluents. They may be prepared e.g in the form of an injectable solution, or in liposomal form.

Particularly preferred in the above indications is the compound of Example 1.

The invention also provides the use of the compounds of the invention for the preparation of pharmaceutical compositions, comprising mixing a compound of the invention with a pharmaceutically acceptable carrier or diluent.

The invention further also provides the compounds of the invention for use as pharmaceuticals, in particular for use as immunomodulators, as antiviral agents and as antiinflammatory agents.

In the following Examples, which illustrate the invention, all temperatures are in degrees centigrade.

### Example 1: 2-deoxy-2-[3-(R)-hydroxytetradecanamido]-3,4-di-0-[3-(R)-hyroxytetradecanoyl]-α-D-glucopyranose-1-phosphate

[R₁,R₂,R₃ = 3-(R)-hydroxytetradecanoyl]
500 mg 2-[3-(R)-benzyloxytetradecanamido]-3,4-di-0-[3-(R)-benzyloxytetradecanoyl]-2-deoxy-6-0-triphenylmethyl-α-D-glucopyranose-1-dibenzyl phosphate are dissolved in 150 ml of tetrahydrofuran/water 4:1 and the resultant mixture is hydrogenated under normal pressure for 12 hours with 500 mg palladium on charcoal. The reaction mixture is filtered and again hydrogenated with 500 mg palladium on charcoal; this operation is repeated two more times (reaction time: 48 hours). The catalyst is filtered off, the tetrahydrofuran is distilled off, the aqueous suspension diluted with 10 ml water and lyophilized. The title compound is obtained:
R_{f} = 0.4 (in chloroform/methanol/water/acetic acid 80:25:5:5
[α]D²⁰ = + 22° (c = 0.1 in chloroform/methanol 3:1).

The mono-tris(hydroxymethyl)aminomethane salt is also obtained.

The starting material is obtained as follows:
a) To a solution of 2 g 2-[3-(R)-benzyloxytetradecanamido]-2-deoxy-6-0-triphenylmethyl-D-glucopyranose in 270 ml of absolute tetrahydrofuran chilled to -60° are added 2.55 ml of a 1.6 M solution of butyllithium in hexane. After 5 minutes a solution of 1.21 g dibenzylphosphorochloridate in 4 ml benzene is added dropwise at the same temperature as above. Agitation is maintained for 20 minutes at -60°, the pH is adjusted to 7 and the solvent evaporated to dryness. The residue is chromatographed without delay (toluene/acetic ester 1:1). 2-[3-(R)-Benzyloxytetradecanamido]-2-deoxy-6-0-triphenylmethyl-α-D-glucopyranose-1-dibenzyl phosphate is obtained (R_{f} = 0.47 in toluene/acetic ester 1:1).
b) To a solution of 1.1 g of the compound obtained as described under a), 1 g 3-(R)-benzyloxytetradecanoic acid and 40 mg of 4-dimethylaminopyridine in 20 ml of dry dichloromethane chilled to 0° are added 619 mg dicyclohexylcarbodiimide. After 30 minutes the reaction mixture is brought to room temperature, after 4 hours the solution is filtered and the solvent evaporated. The residue is chromatographed in toluene/acetic ester 4:1. 2[3-(R)-Benzyloxytetradecanamido]-3,4-di-0-[3-(R)-benzyloxytetradecanoyl]-2-deoxy-6-0-triphenylmethyl-α-D-glucopyranose-1-dibenzyl phosphate is obtained (R_{f} = 0.75 in toluene/acetic ester 4:1).

### Example 2: 2-deoxy-2-[3-(R)-hydroxytetradecanamido]-3-0-[3-(R)-hydroxytetradecanoyl]-4-0-tetradecanoyl-α-D-glucopyranose-1-phosphate

[R₁,R₂ = 3-(R)-hydroxytetradecanoyl; R₃ = tetra decanoyl]
The title compound (R_{f} 0.45 in chloroform/methanol/ water/acetic acid 80:25:5:5) is obtained as described in Example 1, starting from 2-[3-(R)-benzyloxytetradecanamido]-3-0-[3-(R)-benzyloxytetradecanoyl]-2-deoxy-4-0-tetradecanoyl-6-0-triphenylmethyl-α-D-glucopyranose-1-dibenzyl phosphate.

The starting material is obtained as follows:
a) 2.58 g imidazol and 5.03 g 1,3-dichloro-1,1,3,3-tetraisopropyl-disiloxane are stirred for 4 hours at 10° in 65 ml of dimethylformamide. The solution is added dropwise at -10° and under argon to a solution of 5.45 g 2-deoxy-2-[3-(R)-benzyloxytetradecanamido]-D-glucopyranose in 150 ml of dimethylformamide. After 90 minutes about 3 ml of methanol are added, the dimethylformamide is distilled off, the residue taken up in dichloromethane/water, the organic phase dried and distilled off. The residue is chromatographed in toluene/acetic ester 2:1. 2-[3-(R)-Benzyloxytetradecanamido]-2-deoxy-4,6-0-(1,1,3,3-tetraisopropyldisiloxanyliden)-D-glucopyranose is obtained (R_{f} = 0.8 in toluene/acetic ester 1:1).
b) The compound obtained as described under a) above is reacted with butyllithium and dibenzylphosphorochloridate as described in Example 1 a). 2-[3-(R)-Benzyloxytetradecanamido]-2-deoxy-4,6-0-(1,1,3,3-tetraisopropyldisiloxanyliden)-α-D-glucopyranose-1-dibenzyl phosphate is obtained (R_{f} = 0.6 in toluene/acetic ester 1:1).
c) The compound obtained as described under b) above is reacted with 3-(R)-benzyloxytetradecanoic acid as described in Example 1 b). 2-[3-(R)-Benzyloxytetradecanamido]-3-0-[3-(R)-benzyloxytetradecanoyl]-2-deoxy-4,6-0-(1,1,3,3-tetraisopropyldisiloxanyliden)-α-D-glucopyranose-1-dibenzyl phosphate is obtained (R_{f} = 0.6 in toluene/acetic ester 4:1).
d) To a solution chilled to -20° of 800 mg of the compound obtained as described under c) above in 4 ml of dry tetrahydrofuran is slowly added dropwise 168 ml of tetrabutylammonium fluoride in tetrahydrofuran, followed 30 minutes later by 6 µl of acetic acid. The cold reaction mixture is then diluted with 25 ml of dichloromethane and extracted with 20 mg water. The organic phase is dried and evaporated. 2-[3-(R)-Benzyloxytetradecanamido]-3-0-[3-(R)-benzyloxytetradecanoyl]-2-deoxy-α-D-glucopyranose-1-dibenzyl phosphate is obtained without further purification (R_{f} = 0.6 in toluene/acetic ester 3:2).
e) 600 mg of the compound obtained as described under d) above are dissolved into 13 ml of dry dichloromethane and 312 mg trityl chloride and 290 µl of N,N-diisopropylethylamine are added at room temperature. After 18 hours the solvent is distilled off and the residue chromatographed in toluene/acetic ester 4:1. 2-[3-(R)-Benzyloxytetradecanamido]3-0-[3-(R)-benzyloxytetradecanoyl]-2-deoxy-6-0-triphenylmethyl-α-D-glucopyranose-1-dibenzyl phosphate is obtained (R_{f} = 0.6 in toluene/acetic ester 4:1).
f) To a solution of 400 mg of the compound obtained as described under e) above, 69 mg tetradecanoic acid and 10 µg 4-dimethylaminopyridine in 7 ml of dry dichloromethane are added at room temperature 62 mg N,N-dicyclohexylcarbodiimide. After 20 hours the reaction mixture is filtered, the solvent distilled off and the residue chromatographed in toluene/acetic ester 5:1. 2-[3-(R)-Benzyloxytetradecanamido]-3-0-[3-(R)-benzyloxytetradecanoyl]-2-deoxy-4-0-tetradecanoyl-6-O-triphenylmethyl-α-D-glucopyranose-1-dibenzyl phosphate is obtained (R_{f} = 0.75 in toluene/acetic ester 4:1)

### Example 3: 2-deoxy-2-[3-(R)-hydroxytetradecanamido]-3,4-di-O-[3-(R)-tetradecanoyloxytetradecanoyl]-α-D-glucopyranose-1-phosphate

[R₁ = 3-(R)-hydroxytetradecanoyl;
R₂, R₃ = 3-(R)-(tetradecanoyloxy)tetradecanoyl]
The title compound (R_{f} 0.5 in chloroform/methanol/water 10:3:0.1) is obtained as described in Example 1, starting from 2-[3-(R)-benzyloxytetradecanamido]-2-deoxy-3,4-di-0-[3-(R)-tetradecanoyloxytetradecanoyl]-α-D-glucopyranose-1-dibenzyl phosphate.

The starting material is obtained as follows:
a) A solution of 2-[3-(R)-benzyloxytetradecanamido]-2-deoxy-6-O-triphenylmethyl-α-D-glucopyranose-1-dibenzyl phosphate, 3-(R)-tetradecanoyloxytetradecanoic acid and 4-dimethylaminopyridine in dichloromethane is reacted with dicyclohexylcarbodiimide as described in Example 1 b). 2-3[-(R)-Benzyloxytetradecanamido]-2-deoxy-3,4-di-O-[3-(R)-tetradecanoyloxytetradecanoyl]-6-O-triphenylmethyl-α-D-glucopyranose-1-dibenzyl phosphate is obtained (R_{f} = 0.5 in toluene/acetic ester 9:1).
b) 700 mg of the compound obtained as described under a) above are dissolved into 200 ml of diethyl ether and 2.5 ml of formic acid are slowly added dropwise. The reaction mixture is neutralized after 2 hours reaction time with saturated sodium hydrogen carbonate solution. The aqueous phase is separated off and the organic phase washed once with 2 ml of water. The organic phase is dried over magnesium sulfate and evaporated off. The residue is chromatographed over silicagel using toluene/acetic ester 4:1 as an eluent. 2-[3-(R)-Benzyloxytetradecanamido]-2-deoxy-3,4-di-O-[3-(R)-tetradecanoyloxytetradecanoyl]-α-D-glucopyranose-1-di-benzyl phosphate is obtained (R_{f} = 0.4 in toluene/acetic ester 4:1).

## Claims (Claims for the following Contracting State(s): AT, BE, CH, DE, FR, IT, LU, NL, SE, GB)

1. A compound of formula I, 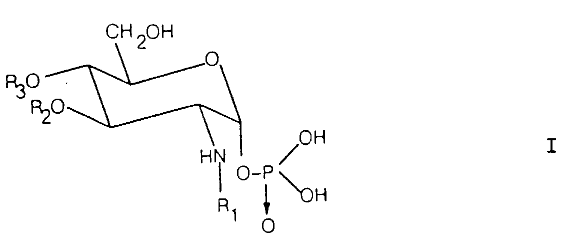 wherein
R₁, R₂ and R₃ independently are alkylcarbonyl of 12 to 16 carbon atoms optionally monosubstituted in the 3 position by hydroxy or alkylcarbonyloxy, whereby the alkylcarbonyl part of alkylcarbonyloxy is of 12 to 16 carbon atoms,
in free form or in salt form.

2. A compound of claim 1 which is 2-deoxy-2-[3-(R)-hydroxytetradecanamido]-3,4-di-0-[3-(R)-hydroxytetradecanoyl]-α-D-glucopyranose-1-phosphate in free form or in salt form.

3. A process for the preparation of a compound of claim 1 in free form or in salt form comprising deprotecting a corresponding compound of formula II, 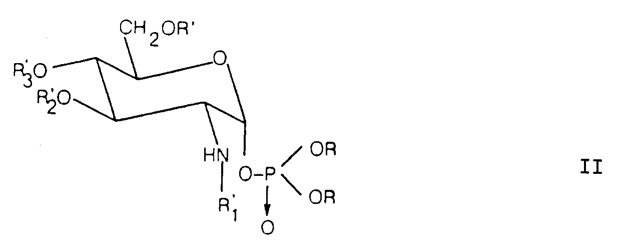 wherein
R and R' are protecting groups and
R₁', R₂' and R₃' have the significances indicated in claim 1 for, respectively, R₁, R₂ and R₃ with the proviso that any hydroxy group therein is in protected form,
and recovering the resultant compound of formula I in free form or in salt form.

4. A pharmaceutical composition comprising a compound of claim 1 in free form or in salt form in association with at least one pharmaceutical carrier or diluent.

5. A compound of claim 1 or 2 for use as a pharmaceutical.

6. A compound of claim 1 or 2 for use as an immunomodulator, as an anti-viral agent or as an anti-inflammatory agent.

## Claims (Claims for the following Contracting State(s): ES, GR)

1. A process for the preparation of a compound of formula I, 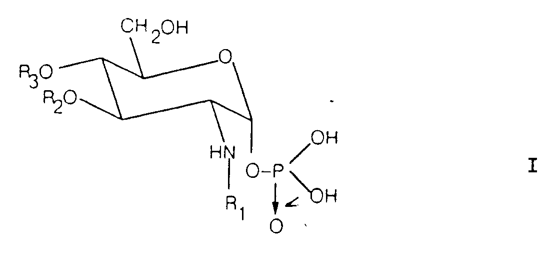 wherein
R₁, R₂ and R₃ independently are alkylcarbonyl of 12 to 16 carbon atoms optionally monosubstituted in the 3 position by hydroxy or alkylcarbonyloxy, whereby the alkylcarbonyl part of alkylcarbonyloxy is of 12 to 16 carbon atoms,
in free form or in salt form,
comprising deprotecting a corresponding compound of formula II, 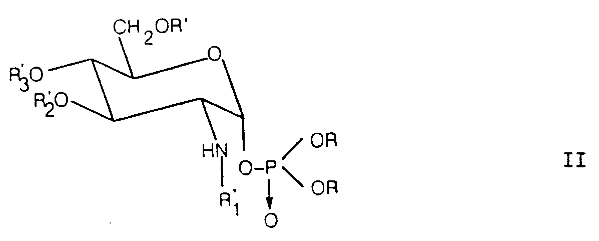 wherein
R and R' are protecting groups and
R₁', R₂' and R₃' have the significances indicated in this claim for, respectively, R₁, R₂ and R₃ with the proviso that any hydroxy group therein is in protected form,
and recovering the resultant compound of formula I in free form or in salt form.

2. A process of claim 1 for the preparation of (2-deoxy-2-[3-(R)-hydroxytetradecanamido]-3,4-di-0-[3-(R)-hydroxytetradecanoyl]-α-D-glucopyranose-1-phosphate in free form or in salt form comprising deprotecting a corresponding compound of formula II wherein
R', R'' and R''' are as defined in claim 1 and
R₁', R₂' and R₃' are 3-(R)-hydroxytetradecanoyl with the hydroxy group therein in protected form,
and recovering the resultant compound in free form or in salt form.

3. A process of claim 1 or 2 for the preparation of the compound of formula I of claim 1 in free form or in salt form comprising deprotecting 2-[3-(R)-benzyloxytetradecanamido]-3,4-di-0-[3-(R)-benzyloxytetradecanoyl]-2-deoxy-6-0-triphenylmethyl-α-D-glucopyranose-1-dibenzyl phosphate and recovering the resultant compound in free form or in salt form.

## Patentansprüche (Patentansprüche für folgende(n) Vertragsstaat(en): AT, BE, CH, DE, FR, IT, LU, NL, SE, GB)

1. Verbindung der Formel I 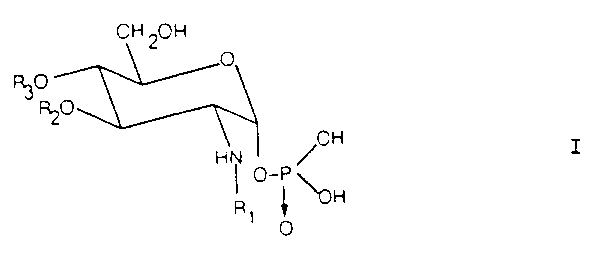 worin R₁, R₂ und R₃ unabhängig Alkylcarbonylreste mit 12 bis 16 Kohlenstoffatomen sind, die gegebenenfalls in Position 3 mit einem Hydroxy- oder Alkylcarbonyloxyrest monosubstituiert sind, wobei der Alkylcarbonylteil des Alkylcarbonyloxyrestes 12 bis 16 Kohlenstoffatome aufweist, in freier Form oder in Salzform.

2. Verbindung nach Anspruch 1 die 2-Deoxy-2-[3-(R)-hydroxytetra-decanamido]-3,4-di-O-[3-(R)-hydroxytetradecanoyl]-α-D-glucopyranose-1-phosphat in freier Form oder in Salzform ist.

3. Verfahren zur Herstellung einer Verbindung nach Anspruch 1 in freier Form oder in Salzform umfassend, daß man bei einer Verbindung der Formel II 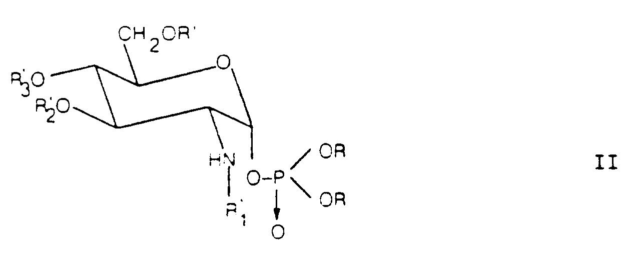 worin R und R' Schutzgruppen sind und R₁', R₂' und R₃' die in Anspruch 1 für R₁, R₂ bzw. R₃ angegebenen Bedeutungen haben, mit dem Vorbehalt, daß jede darin vorliegende Hydroxygruppe in geschützter Form ist, die Schutzgruppen abspaltet und die entstehende Verbindung der Formel I in freier Form oder in Salzform gewinnt.

4. Pharmazeutische Zusammensetzung umfassend eine Verbindung nach Anspruch 1 in freier Form oder in Salzform zusammen mit mindestens einem pharmazeutischen Träger oder Verdünnungsmittel.

5. Verbindung nach Anspruch 1 oder 2 zur Verwendung als Pharmazeutikum.

6. Verbindung nach Anspruch 1 oder 2 zur Verwendung als Immunmodulator, als antivirales Mittel oder als entzündungshemmendes Mittel.

## Patentansprüche (Patentansprüche für folgende(n) Vertragsstaat(en): ES, GR)

1. Verfahren zur Herstellung einer Verbindung der Formel I 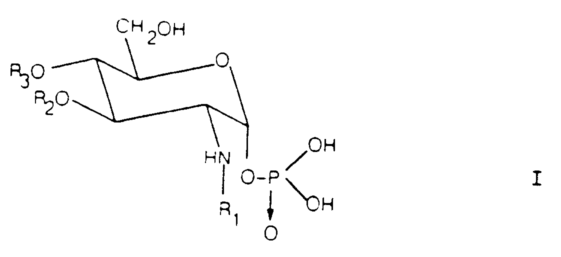 worin R₁, R₂ und R₃ unabhängig Alkylcarbonylgruppen mit 12 bis 16 Kohlenstoffatomen die gegebenenfalls in Position 3 mit einer Hydroxy- oder Alkylcarbonyloxygruppe monosubstituiert sind, wobei der Alkylcarbonylteil der Alkylcarbonyloxygruppe 12 - 16 Kohlenstoffatome aufweist, in freier Form oder in Salzform, umfassend, daß man bei einer Verbindung der Formel II 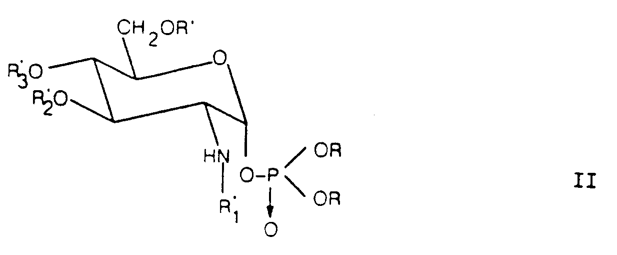 worin R und R' Schutzgruppen sind und R₁', R₂' und R₃' die in diesem Anspruch für R₁, R₂ bzw. R₃ angegebenen Bedeutungen haben, mit dem Vorbehalt, daß jede Hydroxygruppe in geschützter Form ist, die Schutzgruppen abspaltet und die entstehende Verbindung der Formel I in freier Form oder in Salzform gewinnt.

2. Verfahren nach Anspruch 1 zur Herstellung von (2-Deoxy-2-[3-(R)-hydroxytetradecanamido]-3,4-di-O-[3-(R)-hydroxytetradecanoyl]-α-D-glucopyranose-1-phosphat in freier Form oder in Salzform umfassend, daß man bei einer entsprechenden Verbindung der Formel II, worin R', R'' und R''' wie in Anspruch 1 definiert sind und R₁', R₂' und R₃' 3-(R)-Hydroxytetradecanoylgruppen sind, wobei die Hydroxygruppe in geschützter Form Vorliegt, die Schutzgruppen abspaltet und die entsprechende Verbindung in freier Form oder in Salzform gewinnt.

3. Verfahren nach Anspruch 1 oder 2 zur Herstellung der Verbindung der Formel I nach Anspruch 1 in freier Form oder in Salzform umfassend, daß man bei 2-[3-(R)-Benzyloxytetradecanamido]-3,4-di-O-[3-(R)-benzyloxytetradecanoyl]-2-deoxy-6-O-triphenylmethyl-α-D-glucopyranose-1-dibenzyl-phosphat die Schutzgruppen abspaltet und die entstehende Verbindung in freier Form oder in Salzform gewinnt.

## Revendications (Revendications pour l'(les) Etat(s) contractant(s) suivant(s): AT, BE, CH, DE, FR, IT, LU, NL, SE, GB)

1. Un composé de formule I 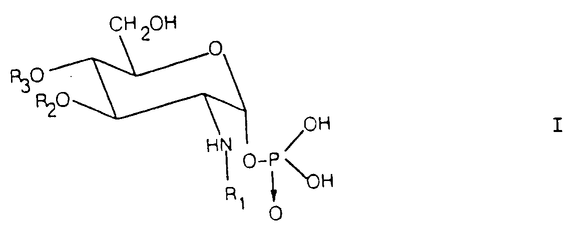 dans laquelle
R₁, R₂ et R₃ signifient,indépendamment les uns des autres, un groupe alkylcarbonyle en C₁₂-C₁₆ éventuellement monosubstitué en position 3 par un groupe hydroxy ou alkylcarbonyloxy, le reste alkylcarbonyle du groupe alkylcarbonyloxy ayant de 12 à 16 atomes de carbone,
sous forme libre ou sous forme de sel.

2. Un composé selon la revendication 1, qui est le 2-désoxy-2-[3-(R)-hydroxy-tétradécanamido]-3,4-di-O-[3-(R)-hydroxytétradécanoyl]-α-D-glucopyranose-1-phosphate sous forme libre ou sous forme de sel.

3. Un procédé de préparation d'un composé selon la revendication 1, sous forme libre ou sous forme de sel, comprenant la déprotection d'un composé correspondant de formule II 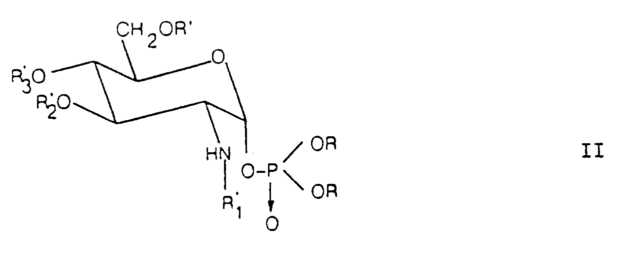 dans laquelle
R et R' signifient des groupes protecteurs, et
R₁', R₂' et R₃' ont les significations indiquées à la revendication 1 respectivement pour R₁, R₂ et R₃, tout groupe hydroxy étant sous forme protégée,
et la récupération du composé résultant de formule I sous forme libre ou sous forme de sel.

4. Une composition pharmaceutique comprenant un composé de la revendication 1, sous forme libre ou sous forme de sel, en association avec au moins un véhicule ou diluant pharmaceutique.

5. Un composé selon la revendication 1 ou 2 pour l'utilisation comme médicament.

6. Un composé selon la revendication 1 ou 2 pour l'utilisation comme immunomodulateur, comme agent anti-viral ou comme agent anti-inflammatoire.

## Revendications (Revendications pour l'(les) Etat(s) contractant(s) suivant(s): ES, GR)

1. Un procédé de préparation d'un composé de formule I 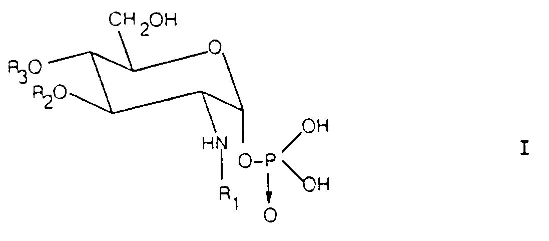 dans laquelle
R₁, R₂ et R₃ signifient,indépendamment les uns des autres, un groupe alkylcarbonyle en C₁₂-C₁₆ éventuellement monosubstitué en position 3 par un groupe hydroxy ou alkylcarbonyloxy, le reste alkylcarbonyle du groupe alkylcarbonyloxy ayant de 12 à 16 atomes de carbone,
sous forme libre ou sous forme de sel,
comprenant la déprotection d'un composé correspondant de formule II 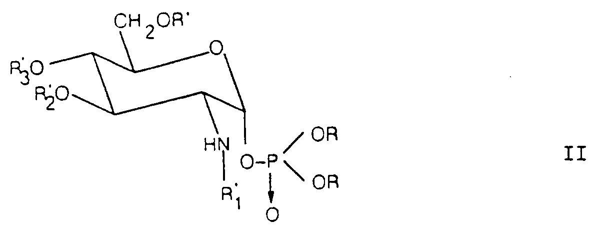 dans laquelle
R et R' signifient des groupes protecteurs, et
R₁', R₂' et R₃' ont les significations indiquées dans la présente revendication respectivement pour R₁, R₂ et R₃, tout groupe hydroxy étant sous forme protégée,
et la récupération du composé résultant de formule I sous forme libre ou sous forme de sel.

2. Un procédé selon la revendication 1 pour la préparation du 2-désoxy-2-[3-(R)-hydroxy-tétradécanamido]-3,4-di-O-[3-(R)-hydroxytétradécanoyl]-α-D- glucopyranose-1-phosphate sous forme libre ou sous forme de sel, comprenant la déprotection d'un composé correspondant de formule II où
R', R'' et R''' sont tels que définis à la revendication 1, et
R₁', R₂' et R₃' signifient un groupe 3-(R)-hydroxytétradécanoyle ayant le groupe hydroxy sous forme protégée,
et la récupération du composé résultant sous forme libre ou sous forme de sel.

3. Un procédé selon la revendication 1 ou 2 de préparation du composé de formule I selon la revendication 1, sous forme libre ou sous forme de sel, comprenant la déprotection du 2-[3-(R)-benzyloxytétradécanamido]-3,4-di-O-[3-(R)-benzyloxytétradécanoyl]-2-désoxy-6-O-triphénylméthyl-α-D-glucopyranose-1-dibenzyl-phosphate et la récupération du composé résultant sous forme libre ou sous forme de sel.
